# EUROPÄISCHE PATENTSCHRIFT

(11) **EP 0 678 294 B1**
(45) Veröffentlichungstag und Bekanntmachung des Hinweises auf die Patenterteilung: **01.07.1998**
(21) Anmeldenummer: 95105621.7
(22) Anmeldetag: 13.04.1995
(51) Int. Cl.: A61K 7/50, A61K 7/08, A61K 7/06

(54) **Mittel und Verfahren zum Reinigen von menschlichen Haaren sowie Verwendung eines Mittels hierfür**
Agent and method for cleaning human hair as well as use of such an agent
Agent et procédé de nettoyage des cheveux humains et utilisation d'agent à cet effet

(30) Priorität: 18.04.1994 DE 4413430
(43) Veröffentlichungstag der Anmeldung: 25.10.1995
(73) Patentinhaber: GOLDWELL GmbH, D-64297 Darmstadt (DE)
(72) Erfinder: Weissbäcker, Sylvia, D-64807 Dieburg (DE); Kameda, Takuo, Funabashishi, 274 Chiba (JP); Nomura, Tadeshi, Dr., D-65936 Frankfurt (DE)

(56) Entgegenhaltungen:
- EP-A- 0 061 701
- EP-A- 0 348 015
- WO-A-92/00719
- US-A- 3 990 991
- US-A- 4 832 872

## Beschreibung

Die vorliegende Erfindung betrifft ein neues lagerstabiles Mittel sowie ein Verfahren zum Reinigen von menschlichen Haaren und die Anwendung dieses Mittels hierzu.

Haarwaschmittel zum Reinigen von Haaren, die in der Regel ein Tensid oder insbesondere Tensid-Gemische und die verschiedenartigsten Zusätze und Wirkstoffe enthalten, sind seit Urzeiten bekannt und bewährt.

In der EP-A-0348015 wird beispielsweise ein Haarreinigungsmittel offenbart, welches ein Öl absorbierende Substanz und ein Cellulosederivat enthält.

Trotz aller Vielfalt der für diesen Zweck bereits vorgeschlagenen Zusammensetzungen besteht jedoch immer noch ein Bedürfnis nach Haarreinigungsmitteln, die, ohne das Haar und die Kopfhaut anzugreifen, in der Lage sind, auch für eine möglichst vollständige Entfernung der durch die Kopfhaut, insbesondere deren Talgdrüsen abgesonderten, öligen und fettigen Bestandteile, des Sebums, zu sorgen und insbesondere auch ein zu starkes und schnelles Nachfetten der Haare zu verhindern.

Es wurde nunmehr gefunden, und dies ist Gegenstand der vorliegenden Erfindung, daß eine Lösung dieser Aufgabe, ein verbessertes Mittel zum Reinigen menschlicher Haare und ein Verfahren unter Verwendung dieses Mittels zur Verfügung zu stellen, darin besteht, daß man auf die Haare eine Zusammensetzung aufbringt, die in wäßriger Grundlage eine Kombination aus
a) etwa 1 bis etwa 20 Gew.-% mindestens eines anionischen, nichtionischen, amphoteren und/oder zwitterionischen Tensids;
b) etwa 0,25 bis etwa 7,5 Gew.-% mindestens eines wasserunlöslichen, teilchenförmigen, synthetischen, vernetzten Copolymeren aus einem C₁-C₁₈-Alkylmethacrylat, mindestens einem weiteren Monomeren mit mehr als einer polymerisierbaren Doppelbindung, und, gegebenenfalls, weiteren ungesättigten polymerisierbaren Verbindungen; und
c) etwa 0,25 bis etwa 7,5 Gew.-% mindestens einer Verbindung, ausgewählt aus der Gruppe Hydroxy-C₁-C₄-alkylcellulose(n), Carboxymethyl- und Carboxyethylcellulose und deren wasserlöslichen Salzen, und/oder gegebenenfalls neutralisierten carboxylgruppenhaltigen Polymeren,
   wobei die Prozentsätze jeweils auf die Gesamtzusammensetzung bezogen sind, enthält.

Die Anwendung des Mittels erfolgt in der Weise, daß es auf das Haar aufgebracht und nach etwa ein- bis fünfzehnminütiger Einwirkung wieder ausgespült wird.

Dabei kann das zu behandelnde Haar entweder trocken oder angefeuchtet sein, gegebenenfalls ist auch eine Vorbehandlung mit einem konventionellen Shampoo zweckmäßig. Ebenso kann das Haar nach der Entfernung des erfindungsgemäßen Reinigungsmittels und anschließendem Spülen mit Wasser sofort getrocknet oder, falls gewünscht, ebenfalls nochmals mit einem konventionellen Haarwaschmittel shampooniert werden.

Durch diese Behandlung wird eine praktisch vollständige Entfernung des Sebums, d.h. der von den Talgdrüsen abgesonderten fettigen und öligen Bestandteile, auf dem Haar erreicht; die Nachfettung des Haares erfolgt deutlich verzögert, so daß das unschöne, fettige Aussehen für längere Zeit unterbunden ist.

Die erfindungsgemäßen Zusammensetzungen enthalten etwa 1 bis etwa 20 Gew.-%, vorzugsweise etwa 2,5 bis etwa 15, insbesondere etwa 3 bis etwa 6 Gew.-%, mindestens eines anionischen, nichtionischen, amphoteren und/oder zwitterionischen Tensids.

Bevorzugt sind hierbei insbesondere anionische und nichtionische Tenside, vorzugsweise im Gemisch untereinander.

Geeignete anionische Tenside sind insbesondere die bekannten C₁₀-C₁₈-Alkylethersulfate bzw. deren wasserlöslichen Salze, beispielsweise C₁₂-C₁₄-Alkylethersulfat und Laurylethersulfat, insbesondere mit 1 bis 10, vorzugsweise 2 bis 5 Ethylenoxidgruppen im Molekül, weiterhin entsprechende Fettsäureamidsulfate, die durch Ethoxylierung und anschließende Sulfatierung von Fettsäurealkanolamiden erhalten wurden, langkettige Acylsarcosinate bzw. deren Alkalisalze, α-Olefinsulfonate bzw. deren Salze, Alkalisalze von Sulfobernsteinsäurehalbestern, beispielsweise das Dinatriumsalz des Monooctylsulfosuccinats und Alkalisalze langkettiger Monoalkylethoxysulfosuccinate (z.B. mit 2 bis 6 EO-Einheiten), Isethionate, Salze langkettiger Mono- und Dialkylphosphate sowie insbesondere auch langkettige Alkylethercarbonsäuren und deren Alkalisalze, die milde, hautverträgliche Detergentien darstellen.

Obwohl im Prinzip ebenfalls zum Einsatz geeignet, sind die bekannten C₁₀-C₁₈-Alkylsulfate und deren Salze weniger beliebt, da sie sich als nicht so hautmild erwiesen haben wie die vorstehend aufgezählten.

Eine Übersicht über geeignete anionaktive Tenside findet sich in den einschlägigen Monographien, beispielsweise bei K.Schrader, "Grundlagen und Rezepturen der Kosmetika", 2. Aufl. (1989, Hüthig Buchverlag), S. 683 bis 691.

Im Gemisch mit anderen anionischen Tensiden können auch Eiweiß-Fettsäure-Kondensationsprodukte an sich bekannter Struktur, insbesondere in Mengen zwischen etwa 0,5 und 5, vorzugsweise 1 bis 3 Gew.-% der Gesamtzusammensetzung, miteingesetzt werden.

Als nichtionische Tenside sind insbesondere die Alkylpolyglucoside, vorzugsweise solche mit einer Alkylgruppe mit 8 bis 18 Kohlenstoffatomen, einem Saccharidrest mit 5 bis 6 Kohlenstoffatomen und einem Polymerisationsgrad zwischen 1 und 2,5, insbesondere zwischen 1,2 und 1,8, geeignet.

Diese Alkylpolyglucoside sind in letzter Zeit insbesondere als ausgezeichnet hautverträgliche schaumverbessernde Mittel in flüssigen Wasch- und Körperreinigungsmitteln bekanntgeworden. Sie können in den erfindungsgemäßen Zusammensetzungen vorzugsweise im Gemisch mit anionaktiven Tensiden eingesetzt werden, derartige Gemische sind beispielsweise aus der EP-A 70 074 bekannt. Weitere geeignete Gemische sind die aus der EP-A 358 216 bekannten Gemische aus Alkylpolyglucosiden und Sulfosuccinaten.

Obwohl Alkylpolyglucoside als nichtionische Tenside in den erfindungsgemäßen Zusammensetzungen bevorzugt werden, ist es auch möglich, stattdessen oder, vorzugsweise gemeinsam mit denselben, weitere nichtionische Tenside einzusetzen.

Als solche sind insbesondere Aminoxide, beispielsweise Lauryldimethylaminoxid oder Cocosdi(hydroxyethyl)aminoxid, geeignet.

Weitere geeignete nichtionische Tensidbestandteile sind beispielsweise langkettige Fettsäuremono- und -dialkanolamide, wie Cocosfettsäuremonoethanolamid und Myristinfettsäuremonoethanolamid und die verschiedenen Sorbitanester, beispielsweise Polyethylenglycolsorbitanstearinsäureester, Fettsäurepolyglycolester oder auch Mischkondensate aus Ethylenoxid und Propylenoxid, wie sie beispielsweise unter der Handelsbezeichnung "Pluronics" im Verkehr sind.

Die erfindungsgemäßen Zusammensetzungen können auch amphotere bzw. zwitterionische Tenside enthalten, alleine oder vorzugsweise in einer Menge von etwa 0,5 bis etwa 2,5 Gew.-%, bezogen auf die Gesamtzusammensetzung, im Gemisch mit anionischen und/oder nichtionischen Tensiden.

Geeignete amphotere bzw. zwitterionische oberflächenaktive Substanzen sind insbesondere die verschiedenen Betaine wie Fettsäureamidoalkylbetaine und Sulfobetaine, beispielsweise Laurylhydroxysulfobetain. Auch langkettige Alkylaminosäuren sind geeignet. Eine Aufzählung geeigneter amphoterer bzw. zwitterionischer Tenside findet sich bei Schrader, l.c., S.598 bis 600.

Der zweite essentielle Bestandteil der erfindungsgemäßen Zusammensetzung, der in einer Menge von etwa 0,25 bis etwa 7,5 Gew.-%, insbesondere 0,5 bis etwa 5 Gew.-%, besonders bevorzugt in einer Menge zwischen etwa 1 und etwa 3 Gew.-%, jeweils berechnet auf die Gesamtzusammensetzung, enthalten ist, ist ein synthetisches, vernetztes Copolymerisat aus einem C₁-C₁₈-Alkylmethacrylat, mindestens einem weiteren Monomeren mit mehr als einer polymerisierbaren Doppelbindung und gegebenenfalls weiteren ungesättigten polymerisierbaren Verbindungen.

Als geeignete Alkylmethacrylate sind insbesondere diejenigen mit einer Alkylgruppe mit 5 bis 18 Kohlenstoffatomen zu nennen, insbesondere 2-Ethylhexylmethacrylat, Decylmethacrylat, Laurylmethacrylat, Myristylmethacrylat und Stearylmethacrylat; jedoch sind im Prinzip auch niedere Methacrylate, insbesondere Methylmethacrylat, Ethylmethacrylat, n-Propyl- und Isopropylmethacrylat, n-Butyl- und Isobutylmethacrylat geeignet.

Als Comonomere, die mindestens 2 polymerisierbare Doppelbindungen aufweisen, sind insbesondere Dioldimethacrylate, beispielsweise Ethandioldimethacrylat, Diethylenglycoldimethacrylat, Triethylenglycoldimethacrylat und Tetraethylenglycoldimethacrylat geeignet, jedoch können auch höhere Polyalkoholdimethacrylate oder Divinylbenzol, Allylmethacrylat, Diallylitaconat, etc. eingesetzt werden.

Als weitere Monomere können in untergeordneten Mengen solche eingesetzt werden, die die Hydrophobie der Copolymerisate nicht negativ beeinflussen, beispielsweise Styrol, α-Methylstyrol, Vinylacetat, etc.

Ein bevorzugtes vernetztes Copolymeres im Sinne der Erfindung ist ein solches aus Laurylmethacrylat und Ethandioldimethacrylat, vorzugsweise in einem molaren Verhältnis von 2 : 1.

Geeignete vernetzte Copolymerisate sind in der US-A 4 724 240 beschrieben, auf deren Offenbarung hier ausdrücklich Bezug genommen wird.

Die bevorzugte Einzelpartikelgröße der erfindungsgemäß eingesetzten hydrophoben teilchenförmigen vernetzten Copolymeren liegt vorzugsweise unterhalb 1 Micron, diese Microteilchen verdichten sich zu Agglomeraten im Bereich zwischen etwa 5 und etwa 200, vorzugsweise 10 bis etwa 100, insbesondere 20 bis 80 Micron. Diese Teilchen können bei der Anwendung zu Makropartikeln im Bereich zwischen etwa 200 und etwa 1500 Micron agglomerieren.

Ein im Rahmen der Erfindung besonders bevorzugtes vernetztes synthetisches teilchenförmiges hydrophobes Copolymerisat wird unter der Bezeichnung "Polytrap^{R} Q 5-6603" von der Firma Dow Corning Co. vertrieben; auf die einschlägigen Informationsschriften dieses Unternehmens zu diesem Produkt wird verwiesen.

Die im Rahmen der Erfindung geeigneten hydrophoben vernetzten Polymerteilchen lassen sich auch anhand ihrer ölabsorbierenden Eigenschaften dergestalt definieren, daß sie eine Squalen-Absorption "von mindestens 0,5 ml/g" aufweisen. Diese Konstante ist in der EP-B 348 015 definiert, wo auch die entsprechende Bestimmungsmethode beschrieben ist.

Der dritte essentielle Bestandteil der erfindungsgemäßen Zusammensetzung ist mindestens eine Verbindung aus der Gruppe der Hydroxy-C₁-C₄-alkylcellulosen, insbesondere Hydroxyethylcellulose, Hydroxypropylcellulose und Hydroxypropylmethylcellulose, wie sie beispielsweise unter den Namen "Klucel", "Methocel", "Viscontran" oder "Cellosize" im Handel sind, sowie auch Carboxymethylcellulose und Carboxyethylcellulose bzw. deren wasserlösliche Salze, insbesondere Natriumcarboxymethylcellulose, sowie gegebenenfalls neutralisierte carboxylgruppenhaltige Polymere, wobei insbesondere die gegebenenfalls vernetzten und/oder neutralisierten Polyacrylsäuren, bekannt unter dem Trivialnamen "Carbomer" und den Handelsnamen "Carbopol^{R}" der Firma B.F. Goodrich Co., im Handel sind.

Diese Substanzen sind in einer Menge zwischen etwa 0,25 und etwa 7,5 Gew.-%, vorzugsweise etwa 0,25 bis etwa 2,5 Gew.-%, in den erfindungsgemäßen Zusammensetzungen enthalten; gemäß einer bevorzugten Ausführungsform werden Gemische aus Hydroxyethylcellulose und/oder Hydroxypropylcellulose und einer (teil-)neutralisierten Polyacrylsäure eingesetzt.

Der Zusatz dieser Komponenten dient insbesondere der Stabilisierung der Zusammensetzung aus Tensid und vernetzten hydrophoben Polymerteilchen, da ohne Zusatz dieser Substanzen lagerstabile Zubereitungen nicht erhalten werden können.

Die erfindungsgemäßen Mittel können selbstverständlich alle in Haarpflege- und -reinigungsmitteln üblichen Stoffe enthalten, soweit diese deren Funktion und Stabilität nicht beeinträchtigen.

Genannt seien beispielsweise Antischuppen-Wirkstoffe, konditionierende Mittel wie anionische, kationische und nichtionische Polymere, Farbstoffe, Parfums, etc.

Zur Vermeidung von Wiederholungen wird auch hierzu auf die bereits zitierte Monographie von Schrader, S.700 bis 707, verwiesen.

Die folgenden Ausführungsbeispiele sollen die erfindungsgemäßen Reinigungsmittel und ihre Anwendung veranschaulichen.

### Beispiel 1

| | |
|---|---|
| Natriumlaurylethersulfat (∼ 2 EO-Einheiten) | 2,3 (Gew.-%) |
| C₉-C₁₁-Alkylpolyglucosid (P.G.: < 1,5) | 1,0 |
| Fettsäure(Abietoyl)-Eiweißhydrolysat, Kaliumsalz | 1,0 |
| Polysorbate 20 | 1,0 |
| 1,2-Propylenglykol | 5,0 |
| Eichenrindenextrakt | 1,0 |
| Hydrophobe Polymerteilchen (Ethandioldimethacrylat/Laurylmethacrylat-Copolymerisat; Polytrap^{R} Q5-6603) | 2,0 |
| Polyacrylsäure (Carbomer) | 1,1 |
| Hydroxyethylcellulose | 0,6 |
| Natronlauge, 32%ig | 1,3 |
| Ichthyol, hell | 0,5 |
| Parfum, Konservierungsmittel, Farbstoff | q.s. |
| Wasser | @ 100,0 |

Das Produkt wurde auf trockenes, fettiges Haar aufgebracht und nach etwa zehnminütiger Einwirkungsdauer gespült und mit einem konventionellen Shampoo nachgewaschen.

Gegenüber ausschließlich mit konventionellem Shampoo gewaschenen Haar setzte das Fettwerden das Haares wesentlich später ein.

Dieser Effekt war nach mehrmaliger Applikation noch deutlicher wahrnehmbar, so daß das Intervall zwischen den Haarwäschen verlängert werden konnte.

Bei der Umkehr der Reihenfolge, d.h., Vorwäsche mit einem konventionellen Shampoo und anschließende fünfminütige Behandlung mit dem erfindungsgemäßen Mittel und darauffolgendes Spülen mit Wasser, wurde der gleiche Effekt erreicht.

### Beispiel 2

| | |
|---|---|
| Natriumlaurylethersulfat (∼ 2 EO-Einheiten) | 8,5 (Gew.-%) |
| Dinatriumlaurylethersulfosuccinat (∼ 2,5 EO-Einheiten) | 2,0 |
| C₉-C₁₁-Alkylglucosid (P.G.: < 1,5) | 1,0 |
| Hydrophobe Polymerteilchen (Polytrap^{R} Q 5-6603) | 5,0 |
| Polyacrylsäure (Carbopol^{R} 981) | 0,5 |
| Hydroxyethylcellulose | 0,3 |
| NaOH | 0,1 |
| Parfum, Konservierungsmittel | q.s. |
| Wasser | @ 100,0 |

Das Mittel wurde auf feuchtes Haar aufgebracht und nach zehnbis fünfzehnminütiger Anwendungsdauer mit Wasser ausgespült.

Gegenüber der Verwendung üblicher Shampoos wurde eine deutliche Verringerung des Nachfettens des so behandelten Haares festgestellt.

## Patentansprüche

1. Zusammensetzung zum Reinigen von menschlichen Haaren, enthaltend in wäßriger Grundlage eine Kombination aus
a) etwa 1 bis etwa 20 Gew.-% mindestens eines anionischen, nichtionischen, amphoteren und/oder zwitterionischen Tensids;
b) etwa 0,25 bis etwa 7,5 Gew.-% mindestens eines wasserunlöslichen, teilchenförmigen, synthetischen, vernetzten Copolymeren aus einem C₁-C₁₈-Alkylmethacrylat, mindestens einem weiteren Monomeren mit mehr als einer polymerisierbaren Doppelbindung, und, gegebenenfalls, weiteren ungesättigten polymerisierbaren Verbindungen; und
c) etwa 0,25 bis etwa 7,5 Gew.-% mindestens einer Verbindung, ausgewählt aus der Gruppe Hydroxy-C₁-C₄-alkylcellulose(n), Carboxymethyl- und Carboxyethylcellulose und deren wasserlöslichen Salzen, und/oder gegebenenfalls neutralisierten carboxylgruppenhaltigen Polymeren,
wobei die Prozentsätze jeweils auf die Gesamtzusammensetzung bezogen sind.

2. Zusammensetzung nach Anspruch 1, dadurch gekennzeichnet, daß sie etwa 2,5 bis etwa 15 Gew.-% mindestens eines anionischen und/oder nichtionischen Tensids enthält.

3. Zusammensetzung nach Anspruch 1 und/oder 2, dadurch gekennzeichnet, daß sie etwa 0,5 bis etwa 5 Gew.-% eines Copolymeren aus einem C₅-C₁₈-Alkylmethacrylat und einem Alkandioldimethacrylat enthält.

4. Zusammensetzung nach Anspruch 3, dadurch gekennzeichnet, daß sie etwa 0,5 bis etwa 5 Gew.-% eines Copolymerisats aus Laurylmethacrylat und Ethandioldimethacrylat enthält.

5. Zusammensetzung nach einem oder mehreren der Ansprüche 1 bis 4, dadurch gekennzeichnet, daß sie etwa 0,25 bis etwa 2,5 Gew.-% einer gegebenenfalls vernetzten Polyacrylsäure bzw. eines Salzes derselben enthält.

6. Zusammensetzung nach einem oder mehreren der Ansprüche 1 bis 5, dadurch gekennzeichnet, daß sie etwa 0,25 bis etwa 2,5 Gew.-% Hydroxyethylcellulose und/oder Hydroxypropylcellulose enthält.

7. Verfahren zum Reinigen von menschlichen Haaren, dadurch gekennzeichnet, daß auf gegebenenfalls mit einem konventionellen Shampoo vorgereinigtes Haar eine Zusammensetzung nach einem oder mehreren der Ansprüche 1 bis 6 aufgebracht und nach etwa ein- bis fünfzehnminütiger Einwirkung aus dem Haar ausgespült wird.

8. Verwendung einer Zusammensetzung, enthaltend in wäßriger Grundlage eine Kombination aus
a) etwa 1 bis etwa 20 Gew.-% mindestens eines anionischen, nichtionischen, amphoteren und/oder zwitterionischen Tensids;
b) etwa 0,25 bis etwa 7,5 Gew.-% mindestens eines wasserunlöslichen, teilchenförmigen, synthethetischen, vernetzten Copolymeren aus einem C₁-C₁₈-Alkylmethacrylat, mindestens einem weiteren Monomeren mit mehr als einer polymerisierbaren Doppelbindung, und, gegebenenfalls, weiteren ungesättigten polymerisierbaren Verbindungen; und
c) etwa 0,25 bis etwa 7,5 Gew.-% mindestens einer Verbindung, ausgewählt aus der Gruppe Hydroxy-C₁-C₄-alkylcellulose(n), Carboxymethyl- und Carboxyethylcellulose und deren wasserlöslichen Salzen, und/oder gegebenenfalls neutralisierten carboxylgruppenhaltigen Polymeren,
wobei sich die Prozentsätze jeweils auf die Gesamtzusammensetzung beziehen, zur Reinigung von menschlichen Haaren.

## Claims

1. Aqueous composition for cleansing of human hair comprising a combination of
a) about 1% to about 20% by wt. of at least one anionic, nonionic, amphoteric, and (or) zwitterionic surfactant;
b) about 0.25% to about 7.5% by wt. of at least one water-insoluble, particle-shaped, synthetic, cross-linked copolymer comprising a C₁-C₁₈-alkyl methacrylate, at least another monomer having more than one polymerizable double bond, and optionally further unsaturated polymerizable compounds; and
c) about 0.25% to about 7.5% by wt. of at least one compound selected from the group of hydroxy-C₁-C₄-alkyl cellulose(s), carboxymethyl and carboxyethyl celluloses and the water-soluble salts thereof, and (or) optionally neutralized polymers containing carboxylic groups,
whereby all percentages are calculated to the total composition.

2. Composition according to claim 1, characterized in that it comprises about 2.5% to about 15% by wt. of at least one anionic and (or) nonionic surfactant.

3. Composition according to claim 1 and (or) 2, characterized in that it comprises about 0.5% to about 5% by wt. of a copolymer of a C₅-C₁₈-alkyl methacrylate and of an alkandiol dimethacrylate.

4. Composition according to claim 3, characterized in that it comprises about 0.5% to about 5% by wt. of a copolymer of lauryl methacrylate and ethanediol dimethacrylate.

5. Composition according to one or more of the claims 1 to 4, characterized in that it comprises about 0.25% to about 2.5% by wt. of an optionally cross-linked polyacrylic acid or a salt thereof.

6. Composition according to one or more of the claims 1 to 5, characterized in that it comprises about 0.25 to about 2.5% by wt. of hydroxyethyl cellulose and (or) hydroxypropyl cellulose.

7. Process for cleansing of human hair, characterized in that a composition according to one or more of the claims 1 to 6 is applied onto the hair being optionally pre-treated with a customary shampoo, and rinsed from the hair after a processing time of about 1 to 15 minutes.

8. Use of an aqueous composition comprising a combination of
a) about 1% to about 20% by wt. of at least one anionic, nonionic, amphoteric, and (or) zwitterionic surfactant;
b) about 0.25% to about 7.5% by wt. of at least one water-insoluble, particle-shaped, synthetic, cross-linked copolymer of a C₁-C₁₈-alkyl methacrylate, at least one additional monomer with more than one polymerizable double bond, and optionally further unsaturated polymerizable compounds; and
c) about 0.25% to about 7.5% by wt. of at least one compound selected from the group of hydroxy-C₁-C₄-alkyl cellulose(s), carboxymethyl and carboxyethyl cellulose and the water soluble salts thereof, and (or) optionally neutralized polymers containing carboxylic groups,
whereby all percentages are calculated to the total composition, for the cleansing of human hair.

## Revendications

1. Composition pour le lavage de cheveux humains, contenant, dans une base aqueuse, une association de
a) environ 1 à environ 20% en poids d'au moins un tensioactif anionique, non ionique, amphotère et/ou zwitterionique;
b) environ 0,25 à environ 7,5% en poids d'au moins un copolymère synthétique réticulé particulaire, insoluble dans l'eau, à base d'un méthacrylate d'alkyle en C₁-C₁₈, d'au moins un autre monomère ayant plus d'une double liaison polymérisable et, éventuellement, d'autre composés insaturés polymérisables; et
c) environ 0,25 à 7,5% en poids d'au moins un composé choisi parmi une (des) hydroxyalkyl(C₁-C₄)cellulose(s), la carboxyméthylcellulose et la carboxyéthylcellulose et leurs sels solubles dans l'eau, et/ou des polymères contenant des groupes carboxyle, éventuellement neutralisés,
les pourcentages étant chacun par rapport à la composition totale.

2. Composition selon la revendication 1, caractérisée en ce qu'elle contient d'environ 2,5 à environ 15% en poids d'au moins un tensioactif anionique et/ou non ionique.

3. Composition selon la revendication 1 et/ou la revendication 2, caractérisée en ce qu'elle contient d'environ 0,5 à environ 5% en poids d'un copolymère à base d'un méthacrylate d'alkyle en C₅-C₁₈ et d'un diméthacrylate d'alcanediol.

4. Composition selon la revendication 3, caractérisée en ce qu'elle contient d'environ 0,5 à environ 5%, en poids d'un copolymère de méthacrylate de lauryle et diméthylacrylate d'éthanediol.

5. Composition selon une ou plusieurs des revendications 1 à 4, caractérisée en ce qu'elle contient d'environ 0,25 à environ 2,5% en poids d'un acide polyacrylique éventuellement réticulé ou d'un sel de celui-ci.

6. Composition selon une ou plusieurs des revendications 1 à 5, caractérisée en ce qu'elle contient d'environ 0,25 à environ 2,5% en poids d'hydroxyéthylcellulose et/ou d'hydroxypropylcellulose.

7. Procédé pour le lavage des cheveux humains, caractérisé en ce que l'on applique sur le cheveu, éventuellement prélavé avec un shampooing traditionnel, une composition selon une ou plusieurs des revendications 1 à 6, et, après l'avoir laissée agir pendant 1 à 15 minutes, on l'élimine par rinçage du cheveu.

8. Utilisation d'une composition contenant, dans une base aqueuse, une association de
a) environ 1 à environ 20% en poids d'au moins un tensioactif anionique, non ionique, amphotère et/ou zwitterionique;
b) environ 0,25 à environ 7,5% en poids d'au moins un copolymère synthétique réticulé particulaire, insoluble dans l'eau, à base d'un méthacrylate d'alkyle en C₁-C₁₈, d'au moins un autre monomère ayant plus d'une double liaison polymérisable et, éventuellement, d'autre composés insaturés polymérisables; et
c) environ 0,25 à 7,5% en poids d'au moins un composé choisi parmi une (des) hydroxyalkyl(C₁-C₄)cellulose(s), la carboxyméthylcellulose et la carboxyéthylcellulose et leurs sels solubles dans l'eau, et/ou des polymères contenant des groupes carboxyle, éventuellement neutralisés,
les pourcentages étant chacun par rapport à la composition totale, pour le lavage de cheveux humains.
